# EUROPÄISCHE PATENTSCHRIFT

(11) **EP 1 922 004 B1**
(45) Veröffentlichungstag und Bekanntmachung des Hinweises auf die Patenterteilung: **17.06.2009**
(21) Anmeldenummer: 06776109.8
(22) Anmeldetag: 01.07.2006
(51) Int. Cl.: A61B 17/88, B25B 23/10

(54) **SCHRAUBENDREHER FÜR KNOCHENSCHRAUBEN**
SCREWDRIVER FOR BONE SCREWS
TOURNEVIS POUR VIS A OS

(30) Priorität: 09.09.2005 DE 102005044445; 09.12.2005 DE 102005058868
(43) Veröffentlichungstag der Anmeldung: 21.05.2008
(73) Patentinhaber: Aesculap AG, 78532 Tuttlingen (DE)
(72) Erfinder: SCHUMACHER, Jörg, 78532 Tuttlingen (DE); KRAMER, Ulrich, CH-8374 Oberwangen (CH)
(74) Vertreter: Hoeger, Stellrecht & Partner Patentanwälte
(86) Internationale Anmeldenummer: PCT/EP2006/006423
(87) Internationale Veröffentlichungsnummer: WO 2007/031132

(56) Entgegenhaltungen:
- DE-A1- 10 042 424
- DE-C1- 3 539 502
- US-A- 2 701 494
- US-A- 3 604 487
- US-A1- 2005 092 142

## Beschreibung

Die Erfindung betrifft einen Schraubendreher für Knochenschrauben mit einem Griffteil und einem daran drehfest gehaltenen Schaft, dessen freies Ende einen unrunden Querschnitt aufweist und formschlüssig in eine unrunde Aufnahmeöffnung im Kopf einer Knochenschraube einsetzbar ist, wobei im Schaft mindestens ein Spreizelement in Längsrichtung verschiebbar gelagert ist, welches im Bereich des freien Endes des Schaftes bei seiner Verschiebung in Längsrichtung an einer Aufgleitfläche derart entlanggleitet, daß es über die Kontur des Schaftes seitlich hervortritt.

Ein solcher Schraubendreher ist in der DE 100 42 424 C2 (Basis für den Oberbegriff des Anspruchs 1) beschrieben. Durch Vorschieben des Spreizelementes gelingt es bei diesem Schraubendreher, den Schaft in einfacher Weise lösbar mit der Knochenschraube zu verbinden, so daß die Knochenschraube am freien Ende des Schaftes gehalten ist und an die vorgesehene Applikationsstelle bewegt werden kann.

Bei dem bekannten Schraubendreher erfolgt das Vorschieben des Spreizelementes über eine auf einem Gewinde des Schaftes verdrehbar gelagerte Hülse. Es ist Aufgabe der Erfindung, einen gattungsgemäßen Schraubendreher so auszubilden, daß das Vorschieben und Zurückschieben des Spreizelementes gegenüber dem bekannten Schraubendreher vereinfacht wird.

Diese Aufgabe wird bei einem Schraubendreher der eingangs beschriebenen Art erfindungsgemäß dadurch gelöst, daß das mindestens eine Spreizelement mittels eines elastischen Federelementes in Richtung auf das freie Ende des Schaftes vorgespannt ist und gegen die Wirkung des Federelements im Schaft zurückziehbar ist, bis es nicht mehr über die Kontur des Schaftes seitlich hervorsteht.

Durch die Verwendung eines solchen Federelementes wird das mindestens eine Spreizelement, also ein, zwei oder mehr Spreizelemente, selbsttätig in die vorgeschobene Spannstellung verschoben, in welcher der Schaft durch das mindestens eine Spreizelement formschlüssig und kräftig gegen die Innenwand der Aufnahmeöffnung der Knochenschraube gedrückt wird. Es erfolgt also selbsttätig eine Verbindung zwischen Schraubendreher und Knochenschraube. Zur Lösung dieser Verbindung muß lediglich das mindestens eine Spreizelement gegen die Wirkung des elastischen Federelementes zurückgezogen werden. Der Operateur kann also in einfacher Weise die Verbindung lösen und sie auch in ebenso einfacher Weise wieder dadurch herstellen, daß er das elastische Federelement freigibt, das dann selbsttätig das mindestens eine Spreizelement in die vorgeschobene Klemmstellung verschiebt.

Günstig ist es, wenn ein die Vorschubbewegung des mindestens einen Spreizelements in Richtung auf das freie Ende des Schaftes begrenzender Anschlag vorgesehen ist, so daß die Vorschubbewegung des mindestens einen Spreizelements begrenzt ist, wenn der Schaft nicht in die Aufnahmeöffnung einer Knochenschraube eingreift.

Gemäß einer bevorzugten Ausführungsform ist vorgesehen, daß das mindestens eine Spreizelement über ein Übertragungsglied mit einer Vorschubeinrichtung verbunden ist, die sich im Griffteil befindet, und daß das Federelement Teil der Vorschubeinrichtung ist. Auf diese Weise kann der Operateur im Bereich des Griffelementes das Spreizelement zurückziehen oder freigeben, also die Verbindung zwischen Knochenschraube und Schraubendreher lösen beziehungsweise festlegen.

Günstig ist es, wenn das Übertragungsglied mindestens einen Stab umfaßt, der in mindestens eine seitlich offene, sich über den gesamten Schaft bis zum Griffteil erstreckende Längsnut eingelegt ist. Es können ein, zwei oder mehr Stäbe vorgesehen sein, ebenso ein, zwei oder mehr Längsnuten.

Gemäß einer bevorzugten Ausführungsform umfaßt die Vorschubeinrichtung eine mit dem mindestens einen Spreizelement verbundene, auf dem Schaft verschiebbare Hülse. Durch Verschiebung der Hülse auf dem Schaft kann somit das mindestens eine Spreizelement in die Freigabestellung zurückgezogen werden.

Günstig ist es, wenn die Hülse seitlich vorstehende Griffflächen trägt, die das Zurückziehen erleichtern, insbesondere können diese durch einen radial abstehenden Ringflansch gebildet werden.

Bei einer abgewandelten Ausführungsform ist zur Verschiebung der Hülse ein Schwenkelement vorgesehen, welches an einem Ende schwenkbar am Schaft gelagert ist und um diese Lagerstelle gegen den Schaft verschwenkbar ist, wobei das Schwenkelement einen Mitnehmer aufweist, der beim Verschwenken des Schwenkelementes gegen den Schaft die Hülse entgegen der Wirkung des Federelementes verschiebt. Der Benutzer kann damit durch Betätigung dieses Schwenkelementes die Hülse verschieben und damit das Spreizelement zurückziehen.

Dieses Schwenkelement kann als einfacher Schwenkhebel ausgebildet sein, besonders vorteilhaft ist jedoch eine Ausbildung, bei der das Schwenkelement als die Hülse umgebende Schwenkhülse ausgebildet ist. Eine solche Schwenkhülse umgibt die Hülse allseits und kann in jeder Richtung gegen den Schaft verschwenkt werden, so daß der Benutzer unabhängig von der Winkellage des Schraubendrehers diese Verschwenkung vornehmen kann.

Günstig ist es, wenn das Schwenkelement mittels einer elastisch verformbaren Halterung am Schaft gehalten ist und die Lagerstelle durch ein Abstützelement des Schwenkelementes am Schaft gebildet wird. Die Lagerstelle wird also allein durch die Anlage des Abstützelementes am Schaft oder einem fest damit verbundenen Teil gebildet, eine Fixierung der Schwenkhülse am Schaft oder an einem damit verbundenen Teil erfolgt dagegen über die elastisch verformbare Halterung.

Diese kann vorteilhafterweise durch einen den Schaft konzentrisch umgebenden Balg gebildet werden.

Bei einer bevorzugten Ausführungsform wird das Abstützelement durch einen an der Innenseite der Schwenkhülse nach innen vorspringenden Ringflansch gebildet. Dieser kann gemäß einer besonders bevorzugten Ausführungsform gleichzeitig auch den Mitnehmer bilden, der bei Verschwenkung der Schwenkhülse die Hülse und damit das Spreizelement auf dem Schaft verschiebt.

Bei geeigneter Ausbildung kann die Rückstellkraft des Balges beim Verschwenken der Schwenkhülse so stark sein, daß dieser Balg allein das elastische Federelement ausbildet, welches das Spreizelement in der Ruhestellung in Richtung auf das freie Ende des Schaftes vorspannt.

Besonders vorteilhaft ist es, wenn die Hülse den Schaft im Abstand umgibt und wenn in einem Ringraum zwischen Hülse und Schaft als Federelement eine den Schaft umgebende, sich an dem Schaft und an dem mindestens einen Spreizelement abstützende Schraubenfeder angeordnet ist. Man erhält dann eine sehr platzsparende und kompakte Anordnung zum Zurückziehen und zum selbsttätigen Spannen des mindestens einen Spreizelements.

Gemäß einer besonders bevorzugten Ausführungsform ist vorgesehen, daß die mindestens eine Längsnut und der mindestens eine Stab so dimensioniert sind, daß der mindestens eine Stab zumindest im Bereich des freien Endes des Schaftes spielfrei in die mindestens eine Längsnut eingreift. Dadurch wird sichergestellt, daß im Anlagebereich des Schaftes an der Innenwand der Aufnahmeöffnung in der Knochenschraube keine Freiräume für den Schaft verbleiben, in die das Material des Schaftes bei mechanischer Beanspruchung fließen könnte, die durch ein solches Fließen bedingte Verformung des Schaftes wird dadurch mit Sicherheit vermieden.

Beispielsweise können bei einer bevorzugten Ausführungsform die mindestens eine Längsnut und der mindestens eine Stab zumindest im Bereich des freien Endes des Schaftes zum Nutboden hin konvergierende, ebene Seitenflächen gleicher Neigung aufweisen. Der mindestens eine Stab liegt dann mit diesen geneigten Seitenflächen an den in gleicher Weise geneigten Seitenflächen der mindestens einen Längsnut flächig an, so daß dadurch Spielfreiheit erreicht wird. Die Seitenflächen der mindestens einen Längsnut können dabei gleichzeitig als Aufgleitfläche dienen, beispielsweise dadurch, daß der Abstand der Seitenflächen der mindestens einen Längsnut voneinander zum freien Ende des Schaftes hin kleiner wird oder aber daß die mindestens eine Längsnut zum freien Ende hin weniger tief in den Schaft eintaucht.

Vorzugsweise ist der mindestens eine Stab dabei zumindest im Bereich des freien Ende des Schaftes an seiner dem Nutboden zugewandten Unterseite breiter als der Nutboden, so daß beim Einlegen des mindestens einen Stabes in die mindestens eine Längsnut zwischen der Unterseite und dem Nutboden ein Abstand verbleibt. Dadurch ist sichergestellt, daß der mindestens eine Stab immer über die konvergierenden Seitenflächen an den entsprechenden Seitenflächen der mindestens einen Längsnut anliegt.

Es kann vorgesehen sein, daß die Seitenwände der mindestens einen Längsnut zumindest im Bereich des freien Endes des Schaftes über eine Abrundung in den Nutboden übergehen. Dadurch ergibt sich eine Erhöhung der Festigkeit des Schaftes, da scharfe Kanten im Übergangsbereich zwischen den Seitenflächen und dem Nutboden vermieden werden.

Der Nutboden kann dabei zumindest im Bereich des freien Endes des Schaftes einen bogenförmigen Querschnitt aufweisen.

Es ist weiterhin vorteilhaft, wenn auch die Seitenwände des mindestens einen Stabes zumindest im Bereich des freien Endes des Schaftes über eine Abrundung in die Unterseite übergehen. Auch die Unterseite des mindestens einen Stabes kann zumindest im Bereich des freien Endes des Schaftes einen bogenförmigen Querschnitt aufweisen.

Durch das mindestens eine Spreizelement wird der Schaft mit seinem unrunden Querschnitt gegen die Innenwand der Aufnahmeöffnung gedrückt, so daß durch die unrunden Querschnitte von Schaft und Aufnahmeöffnung und den Formschluß zwischen diesen Flächen eine Drehmitnahme möglich wird. Grundsätzlich könnte eine solche Drehmitnahme beispielsweise durch eine regelmäßig Sechseckform für die Aufnahmeöffnung und den Schaft erreicht werden. Allerdings wird bei einer solchen Formgebung der Schaft durch das mindestens eine Spreizelement mit nur einer Fläche des sechseckigen Querschnitts gegen nur eine Fläche der sechseckigen Aufnahmeöffnung gedrückt. Eine bessere Drehmomentübertragung wäre zu erreichen, wenn die Anlage mehrflächig erfolgen könnte. Um dies zu erreichen, ist gemäß einer bevorzugten Ausführungsform der Erfindung vorgesehen, daß der unrunde Querschnitt der Aufnahmeöffnung und der unrunde Querschnitt des freien Endes des Schaftes derart ausgebildet sind, daß der Schaft durch das mindestens eine Spreizelement in zwei voneinander getrennten Bereichen der Aufnahmeöffnung gegen eine Innenwand der Aufnahmeöffnung angedrückt wird. Man erhält dann im Umfangsbereich an drei Stellen einen Kontakt zwischen dem Schaft und der Aufnahmeöffnung, nämlich einmal im Bereich des mindestens einen Spreizelementes und zum anderen im Bereich der zwei voneinander getrennten Bereiche der Aufnahmeöffnung, so daß eine vergrößerte Übertragungsfläche für das Drehmoment zur Verfügung steht.

Insbesondere kann vorgesehen sein, daß die Aufnahmeöffnung die Form eines regelmäßigen symmetrischen Sechsecks oder Achtecks aufweist und der Schaft im wesentlichen komplementär geformt ist, wobei die dem mindestens einen Spreizelement gegenüberliegende Seitenfläche des Schaftes geringfügig radial nach innen zurückgesetzt ist. Der Querschnitt des Schaftes ist also nicht exakt symmetrisch, sondern weicht geringfügig von der Symmetrie ab. Dadurch ist sichergestellt, daß in jedem Falle die zwei Seitenflächen, die an die dem mindestens einen Spreizelement gegenüberliegende Seitenfläche des Schaftes anschließen, an entsprechenden Innenflächen der Aufnahmeöffnung anliegen, die Drehmomentenübertragung erfolgt also nicht nur an der dem mindestens einen Spreizelement gegenüberliegenden Fläche, sondern an den beiden dieser Fläche benachbarten Flächen und am mindestens einen Spreizelement.

Die beschriebenen vorteilhaften Ausgestaltungen des Schraubendrehers, die sich insbesondere auf die spielfreie Lagerung des Stabes in der mindestens einen Längsnut und/ oder die Anlage des Schaftes an der Innenwand der Aufnahmeöffnung in voneinander getrennten Bereichen beziehen, sind besonders vorteilhaft in Kombination mit der federbelasteten Betätigung des mindestens einen Spreizelementes, diese Merkmale können aber auch bei Schraubendrehern Verwendung finden, bei denen das mindestens eine Spreizelement nicht in der beschriebenen Weise durch ein elastisches Federelement in die Klemmstellung verschoben wird, also beispielsweise bei einem Schraubendreher, wie er in der DE 100 42 424 C2 beschrieben ist. Auch die Spielfreiheit der Stablagerung in der Längsnut und die Anlage des Schaftes an getrennten Bereichen der Aufnahmeöffnung sind besonders günstig in Kombination, diese Merkmale können aber auch jeweils für sich alleine an einem Schraubendreher verwirklicht werden. Auch diese Ausgestaltungen sollen im Rahmen der vorliegenden Erfindung erfaßt werden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Schraubendrehers mit federbelastetem Spreizelement und einer Knochenschraube vor dem Einsetzen des Schaftes des Schraubendrehers in eine Aufnahmeöffnung der Knochenschraube;
- Figur 2:: eine Längsschnittansicht eines Teils des Schraubendrehers der Figur 1 nach dem Einsetzen in den Kopf einer Knochenschraube;
- Figur 3:: eine Längsschnittansicht längs Linie 3-3 in Figur 2 mit einem Schaft mit asymmetrischem Sechseckquerschnitt;
- Figur 4:: eine Ansicht ähnlich Figur 3 mit einer Längsnut für das Spreizelement mit geneigten, ebenen Seitenwänden ;
- Figur 5:: eine Ansicht ähnlich Figur 4 mit einer abgerundeten Kontur der Längsnut;
- Figur 6:: eine perspektivische Schnittansicht eines weiteren Ausführungsbeispiels eines Schraubendrehers im Bereich der Vorschubeinrichtung mit einem Betätigungselement in Form einer Schwenkhülse mit entspanntem Federelement und
- Figur 7:: eine Schnittansicht der Vorschubeinrichtung der Figur 6 längs Linie 7-7 mit gespanntem Federelement.

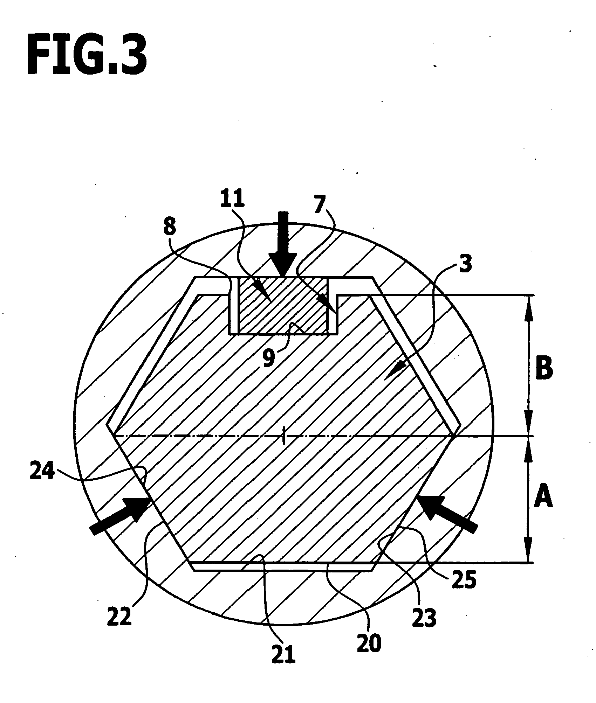

Der in der Zeichnung dargestellte Schraubendreher 1 umfaßt ein Griffteil 2 und einen drehfest in diesem gehaltenen Schaft 3, der an seinem freien Ende 4 den Querschnitt eines Sechsecks aufweist (Figur 3).

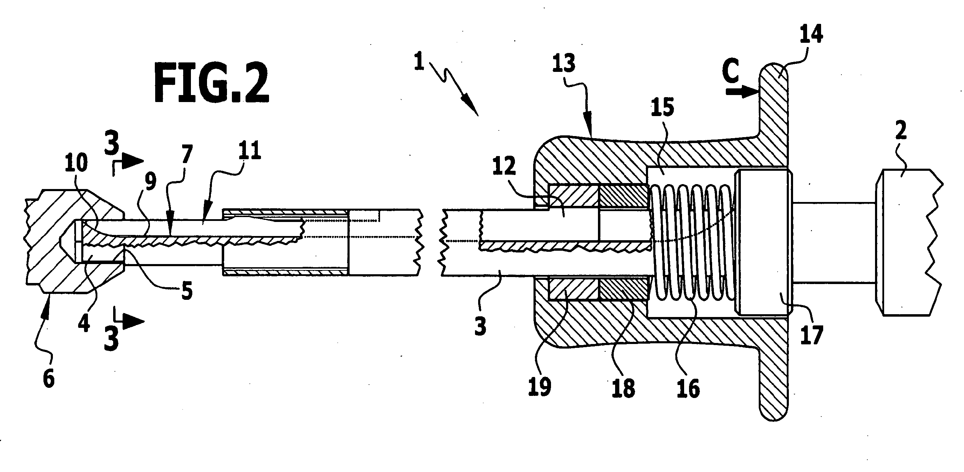

Dieses freie Ende 4 kann in an sich bekannter Weise in eine im wesentlichen komplementär ausgebildete Aufnahmeöffnung 5 einer Knochenschraube 6 eingeführt werden, so daß in diesem Bereich eine drehfeste, formschlüssige Verbindung zwischen Schaft 3 und Knochenschraube 6 entsteht. Im Schaft 3 ist eine sich über den größten Teil seiner Länge erstreckende, zur Seitenfläche hin offene Längsnut 7 angeordnet, deren Breite geringer ist als der Abstand von zwei benachbarten Kanten 8 des sechseckigen Querschnitts am freien Ende 4 des Schaftes 3, wobei sich die Längsnut 7 genau in der Mitte zwischen zwei benachbarten Kanten 8 befindet. Die Tiefe der Längsnut 7 ist etwas kleiner als der halbe Durchmesser des Schaftes 3, der Boden 9 der Längsnut 7 geht im Bereich des freien Endes 4 in eine im Längsschnitt kreisbogenförmige Aufgleitfläche 10 über, die sich tangential an den ebenen Boden 9 anschließt und die bis an die Außenseite des Schaftes 3 ansteigt, so daß die Tiefe der Längsnut 7 am freien Ende 4 des Schaftes 3 auf null abnimmt (Figur 2).

Auf der dem Griffteil 2 benachbarten Seite des Schaftes 3 erstreckt sich die Längsnut 7 bis in das Innere des Griffteiles 2 (Figur 2).

Im Inneren der Längsnut 7 ist ein an die Kontur der Längsnut 7 angepaßter Stab 11 gelagert, der in der Längsrichtung der Längsnut 7 frei verschieblich in dieser geführt ist und der an seinem dem freien Ende 4 des Schaftes 3 benachbarten Ende 12 bogenförmig abgerundet ist, wobei diese bogenförmige Kontur im wesentlichen der bogenförmigen Kontur der Aufgleitfläche 10 entspricht. Im übrigen füllt der Stab 11 die Längsnut im wesentlichen aus und erstreckt sich in Längsrichtung bis kurz vor das Griffteil 2.

Am griffteilseitigen Ende der Längsnut 7 wird der Schaft 3 umgeben von einer Griffhülse 13, die auf dem Schaft 3 in Längsrichtung frei verschieblich ist. Die Griffhülse weist an ihrem dem Griffteil 2 zugewandten Ende einen radial abstehenden Ringflansch 14 auf, der eine ringförmige Grifffläche ausbildet.

Die Griffhülse 13 umgibt den Schaft 3 im Abstand, so daß zwischen der Griffhülse 13 und dem Schaft 3 ein Ringraum 15 ausgebildet wird, in dem eine den Schaft 3 umgebende Schraubenfeder 16 angeordnet ist, die sich einerseits an einem fest mit dem Schaft 3 verbundenen Ringflansch 17 abstützt und andererseits über einen den Schaft 3 umgebenden Ring 18 an einer ebenfalls den Schaft 3 umgebenden ringförmigen Verbreiterung 19 des Stabes 11. Der Stab 11 ist an seinem griffteilseitigen Ende mit dieser Verbreiterung 19 fest verbunden, so daß der Stab 11 durch die Schraubenfeder 16 in Richtung auf das freie Ende 4 des Schaftes 3 verschoben wird.

Die Griffhülse 13 umgreift die Verbreiterung 19 an deren dem Griffteil 2 abgewandten Seite, so daß beim Verschieben der Griffhülse 13 in Richtung auf das Griffteil 2 zu (also in Richtung des Pfeiles C in Figur 2) der Stab 11 in der Längsnut 7 in Richtung auf das Griffteil 2 verschoben wird, dabei wird die Schraubenfeder 16 elastisch komprimiert. Läßt man die Griffhülse 13 wieder los, entspannt sich die Schraubenfeder 16 und verschiebt den Stab 11 wieder in Richtung auf das freie Ende 4 des Schaftes. Die Abmessungen sind dabei so gewählt, daß bei entspannter Schraubenfeder 16 der Stab 11 am freien Ende 4 seitlich aus der Längsnut 7 ausgehoben ist und seitlich über die Kontur des Schaftes vorsteht, während der Stab 11 am freien Ende 4 des Schaftes 3 vollständig in die Längsnut 7 eintritt, wenn die Griffhülse 13 in Richtung auf das Griffteil 2 zurückgezogen und die Schraubenfeder 16 dadurch gespannt ist.

Um das freie Ende 4 des Schaftes 3 in die Aufnahmeöffnung 5 einer Knochenschraube 6 einzusetzen, genügt es für den Operateur, die Griffhülse 13 in Richtung des Pfeiles C zu verschieben und dadurch die Schraubenfeder 16 zu spannen, es ist dann ohne weiteres möglich, das freie Ende des Schaftes 3 in die Aufnahmeöffnung 5 einzuführen, da der Stab 11 vollständig in der Längsnut 7 aufgenommen ist. Sobald der Schaft 3 in die Aufnahmeöffnung 5 eingeführt ist, kann der Operateur die Griffhülse 13 freigeben, dadurch wird unter der Wirkung der sich entspannenden Schraubenfeder 16 der Stab 11 in Richtung auf das freie Ende 4 vorgeschoben, so daß das Ende des Stabes 11 durch die Aufgleitfläche 10 radial nach außen gegen die Innenwand der Aufnahmeöffnung 5 gedrückt wird, das freie Ende des Schaftes 3 wird dadurch in der Aufnahmeöffnung 5 verklemmt.

Man erhält auf diese Weise eine Klemmverbindung zwischen Schraubendreher 1 und Knochenschraube 6, wobei der Schaft 3 im wesentlichen formschlüssig an der Innenwand der Aufnahmeöffnung 5 anliegt.

Wenn der sechseckige Querschnitt des Schaftes 3 und der sechseckige Querschnitt der Aufnahmeöffnung 5 exakt gleich sind, dann würde sich eine exakt formschlüssige Anlage über den gesamten Umfang zwischen Schaft und Aufnahmeöffnung ergeben. Allerdings ist dies in der Praxis nicht möglich, da Fertigungstoleranzen zu beachten sind. Es ist also notwendig, daß die Abmessungen des Schaftes 3 geringfügig kleiner sind als die Abmessungen der Aufnahmeöffnung. Dies führt aber zwangsläufig dazu, daß unter der Wirkung des nach außen gespannten Endes des Stabes 11 die gegenüberliegende Fläche des Schaftes 3 gegen nur eine Seitenfläche der Aufnahmeöffnung 5 gedrückt wird, man erhält also eine Anlage nur auf gegenüberliegenden Seiten der Aufnahmeöffnung.

Um die Anlage zwischen Schaft 3 und Aufnahmeöffnung 5 auch dann zu verbessern, wenn zum Ausgleich von Fertigungstoleranzen ein geringfügiges Spiel vorgesehen sein muß, ist es vorteilhaft, den Querschnitt des Schaftes 3 geringfügig von der Form eines regelmäßigen Sechsecks abweichen zu lassen. Dies erfolgt dadurch, daß die der Längsnut 7 gegenüberliegende Fläche 20 des Schaftes 3 geringfügig radial nach innen gegenüber der Fläche eines regelmäßigen Sechsecks zurückgesetzt ist. Dadurch ergibt sich zwischen dieser Fläche 20 und der direkt gegenüberliegenden Fläche 21 der Aufnahmeöffnung 5 ein geringfügiger Abstand, und dies führt dazu, daß die an die Fläche 20 des Schaftes 3 zu beiden Seiten anschließenden schrägen Flächen 22 und 23 flächig an den direkt an die Fläche 21 der Aufnahmeöffnung 5 anschließenden gleich geneigten Flächen 24 und 25 der Aufnahmeöffnung 5 zur Anlage kommen. Man erhält damit eine großflächigere Anlage zwischen Schaft 3 und Aufnahmeöffnung 5, die Anlage erfolgt in drei getrennten Bereichen, nämlich im Bereich des nach außen gespannten Stabes 11 sowie im Bereich der seitlichen Flächen 22 und 23.

Bei dem in Figur 3 dargestellten Ausführungsbeispiel ist also der regelmäßige sechseckige Querschnitt des Schaftes 3 so abgewandelt, daß die Höhe A des Schaftes auf der der Längsnut 7 gegenüberliegenden Seite geringfügig kleiner ist als die Höhe des Schaftes B auf der Seite der Längsnut 7. Dieser Unterschied kann sehr gering sein, beispielsweise kann er in der Größenordnung von einigen Zehntelmillimetern liegen. Zur Verdeutlichung ist dieser Unterschied in Figur 3 übertrieben dargestellt.

Eine ähnliche Formgebung ist auch möglich, wenn nicht ein Querschnitt eines regelmäßigen Sechseckes gewählt wird, sondern zum Beispiel ein regelmäßiges Achteck.

Bei dem Ausführungsbeispiel der Figur 3 ist die Längsnut 7 im Querschnitt rechteckig ausgebildet, und auch der Stab 11 hat einen rechteckigen Querschnitt. Um auch hier Fertigungstoleranzen auszugleichen, ist es notwendig, daß der Stab 11 in der Längsnut 7 mit Spiel geführt ist, dieses Spiel ist in Figur 3 übertrieben dargestellt. Dieses Spiel hat aber zur Folge, daß beim Übertragen großer Drehmomente das Material des Schaftes 3 sich in den Zwischenraum zwischen dem Schaft 3 und dem Stab 11 hinein verformen kann, das heißt die Stabilität des Schaftes 3 kann leiden.

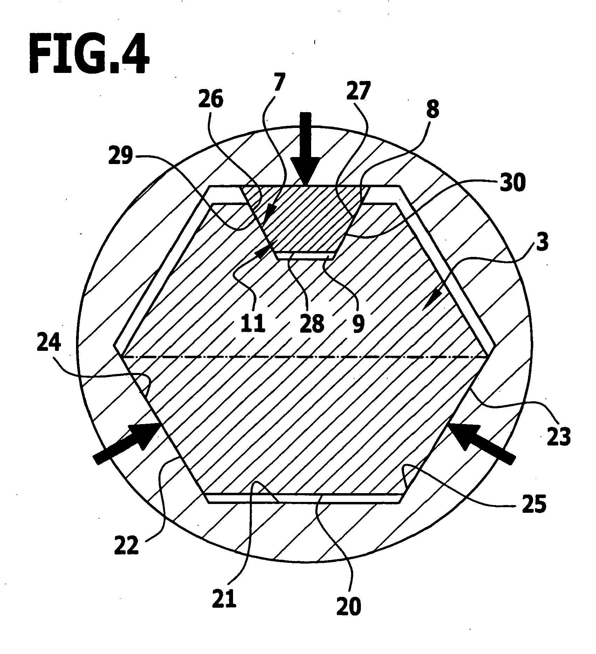

Um dies zu vermeiden, ist in Figur 4 eine abgewandelte Ausführungsform dargestellt. Im Gegensatz zur Ausgestaltung gemäß Figur 3 ist bei der Ausführungsform gemäß Figur 4 der Querschnitt der Längsnut 7 und des Stabes 11 verändert. Die Längsnut 7 weist in diesem Ausführungsbeispiel einen ebenen Boden 9 und zwei zum Boden 9 hin konvergierende, ebene Seitenflächen 26 und 27 auf. Der Winkel kann beispielsweise bei 30° gegenüber der Senkrechten liegen. Der Stab 11 ist ebenfalls an seiner Unterseite 28 eben ausgebildet, an diese ebene Unterseite 28 schließen sich zu beiden Seiten zur Unterseite 28 hin konvergierende ebene Seitenflächen 29 , 30 an. Die Breite der Unterseite 28 ist dabei geringfügig größer als die Breite des Bodens 9 der Längsnut 7, so daß zwischen der Unterseite 28 und dem Boden 9 ein kleiner Abstand verbleibt. Die Seitenflächen 29 und 30 liegen flächig an den Seitenflächen 26 und 27 an, so daß der Stab 11 in der Längsnut 7 spielfrei gelagert ist. Dadurch wird eine Verformung des Schaftes 3 verhindert.

Bei diesem Ausführungsbeispiel kann vorgesehen sein, daß die Seitenflächen 26 und 27 die Aufgleitflächen bilden, die beim Vorschieben des Stabes diesen radial nach außen treiben. Es ist also nicht unbedingt notwendig, daß der Boden 9 nach außen geformt ist, wie dies beim Ausführungsbeispiel der Figur 2 dargestellt ist. Wenn der Abstand der beiden Seitenflächen 26 und 27 zum freien Ende 4 des Schaftes 3 hin kleiner wird, wird der Stab 11 zwangsläufig nach außen geschoben, wenn er in der Längsnut vorgeschoben wird, dasselbe gilt, wenn die Längsnut 7 insgesamt zum freien Ende 4 hin eine geringere Tiefe aufweist.

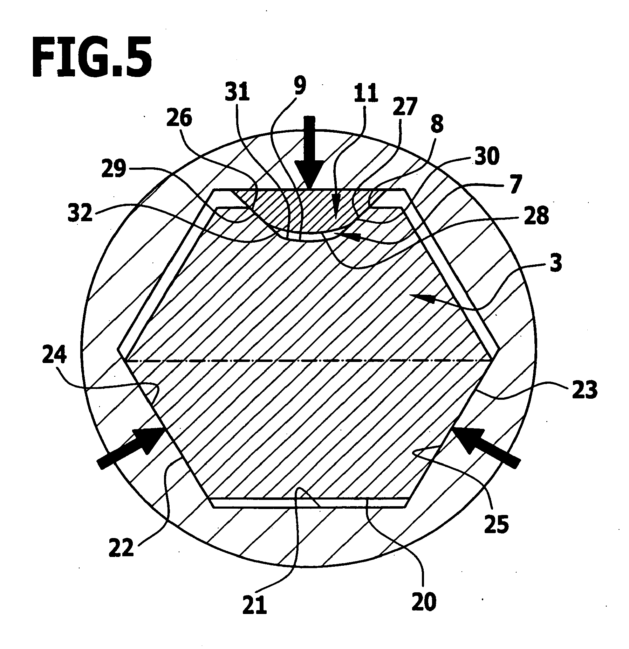

Das Ausführungsbeispiel der Figur 5 entspricht weitgehend dem der Figur 4, es unterscheidet sich von diesem lediglich dadurch, daß die Seitenflächen 26 und 27 nicht längs einer scharfen Kante in den ebenen Boden 9 der Längsnut 7 übergehen, sondern längs einer Abrundung 31, außerdem ist der Boden 9 nicht eben ausgebildet, sondern im Querschnitt bogenförmig. Ebenso ist bei dem Stab 11 vorgesehen, daß die Seitenflächen 29 und 30 über eine Abrundung 32 in die Unterseite 28 übergehen, und auch die Unterseite 28 ist im Querschnitt nicht eben ausgebildet, sondern bogenförmig. Durch diese Ausgestaltung werden scharfe Kanten vermieden, die Festigkeit des Schaftes 3 wird in diesem Bereich dadurch weiter optimiert. Auch in diesem Falle ist eine spielfreie Lagerung des Stabes 11 in der Längsnut 7 gewährleistet.

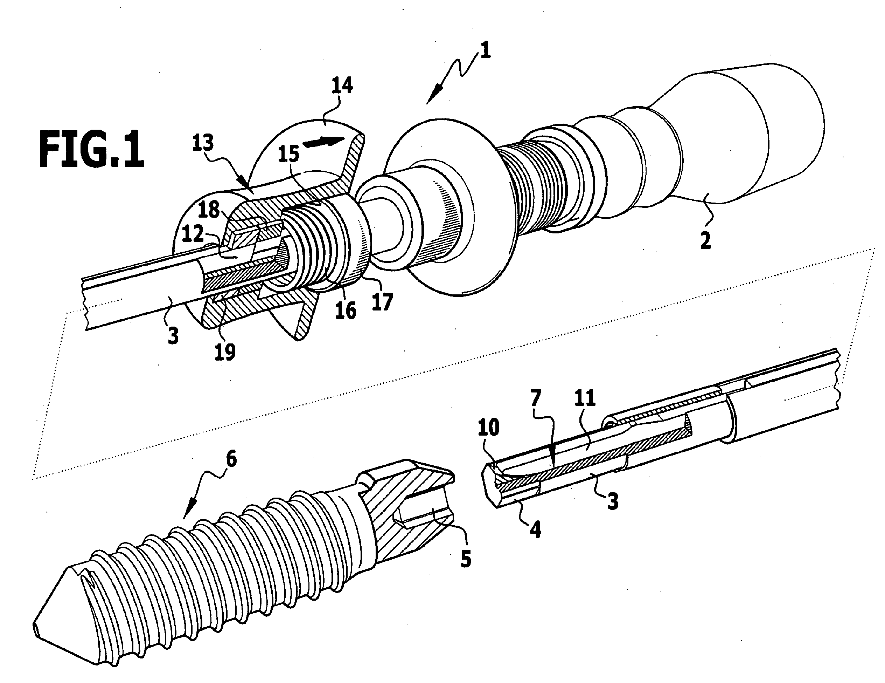

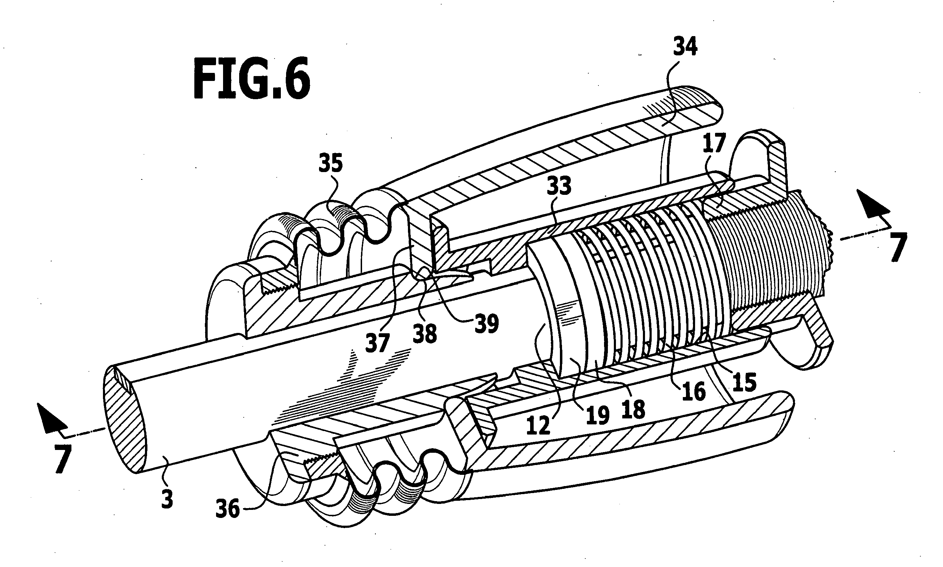

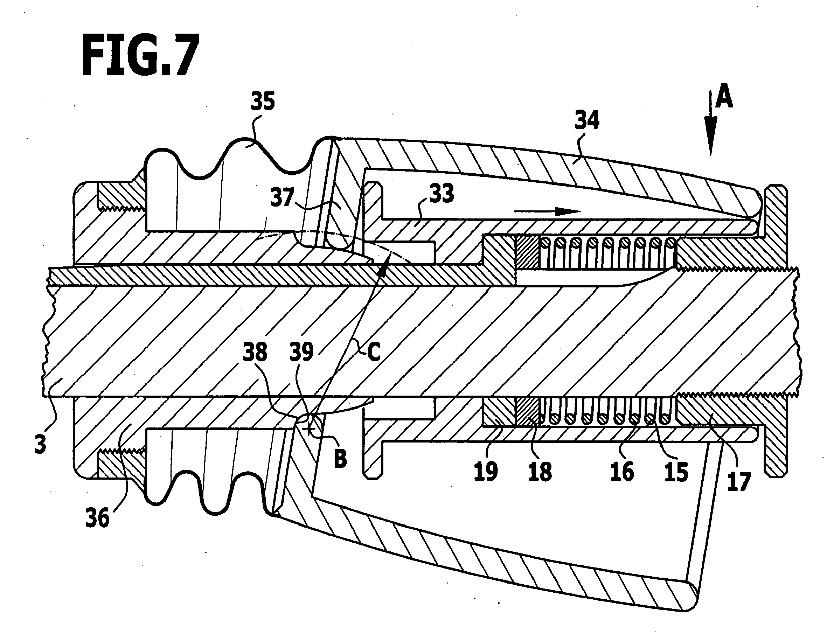

Während bei dem Ausführungsbeispiel der Figuren 1 und 2 der Stab 11 mittels der Griffhülse 13 entgegen der Wirkung der Schraubenfeder 16 verschoben werden kann, ist bei dem abgewandelten Ausführungsbeispiel der Figuren 6 und 7 eine der Griffhülse 13 entsprechende Hülse 33 vorgesehen, die jedoch nicht unmittelbar vom Benutzer über Griffflächen entgegen der Schraubenfeder 16 verschoben werden kann, sondern durch eine die Hülse 33 umgebende Schwenkhülse 34. Diese umgibt die Hülse 33 im Abstand, sie ist an ihrem distalen Ende über einen den Schaft 3 konzentrisch umgebenden elastisch verformbaren Balg 35 mit einem Stützring 36 verbunden, der den Schaft 3 konzentrisch umgibt und mit diesem fest verbunden ist.

Die Schwenkhülse 34 trägt am proximalen Ende des Balges 35 und damit an ihrem distalen Ende einen nach innen radial vorstehenden Ringflansch 37, dessen innere Kante 38 sich an einer Ringschulter 39 des Stützringes 36 abstützt. Diese Abstützung des Ringflansches 37 an der Ringschulter 39 bildet eine Schwenklagerstelle für die Schwenkhülse 34 aus. Wenn die Schwenkhülse 34 an einer Stelle ihres Umfanges gegen den Schaft 3 gedrückt wird, dann führt dies zu einer Verschwenkung der Schwenkhülse 34 um eine derartige Lagerstelle, die dann auf der gegenüberliegenden Seite der Angriffsstelle des Benutzers liegt, wie dies in Figur 7 dargestellt ist. Ein Druck auf den Außenrand der Schwenkhülse 34 in Richtung des Pfeiles A in Figur 7 führt also zu einer Verschwenkung um einen schaftfesten Schwenkpunkt B (Figur 7). Dadurch wird der Ringflansch 37 auf der dem Schwenkpunkt B gegenüberliegenden Seite auf einer Kreisbahn bewegt und von der Ringschulter 39 abgehoben, die Kreisbahn ist in Figur 7 durch den Radius C charakterisiert. Auf der dem Schwenkpunkt B gegenüberliegenden Seite führt dies auch zu einer axialen Verschiebung des Ringflansches 37, und diese axiale Verschiebung überträgt der Ringflansch 37 auf die unmittelbar an ihm anliegende Hülse 33. Diese wird dadurch in proximaler Richtung gegen die Wirkung der Schraubenfeder 16 verschoben, d.h. der Ringflansch 37 wirkt auch als Mitnehmer zur Verschiebung der Hülse 33 und damit zur Verschiebung des Stabes 11.

Der Benutzer kann an jeder Stelle längs des Umfanges der Schwenkhülse 34 diese gegen den Schaft drücken, es wird dann immer auf der gegenüberliegenden Seite ein Schwenkpunkt B ausgebildet, der auf derselben Seite, auf der gegen die Schwenkhülse 34 gedrückt wird, den Ringflansch 37 axial verschiebt und dadurch die Hülse 33.

Bei dem in den Figuren 6 und 7 dargestellten Ausführungsbeispiel wird die Hülse 33 durch die Schraubenfeder 16 wieder in die Ausgangslage zurückgeschoben, dabei wird auch die Schwenkhülse 34 wieder in die Ausgangslage verschwenkt, in der sie konzentrisch zur Hülse 33 angeordnet ist.

Diese Rückbewegung kann durch die elastischen Rückstellkräfte des Balges 35 unterstützt werden. Bei geeigneter Ausbildung des Balges kann auch allein die elastische Rückstellkraft des Balges 35 verwendet werden, um die Hülse 33 in die distale Endstellung zu verschieben, dann ist es allerdings notwendig, daß zwischen der Hülse 33 und dem Ringflansch 37 eine zusätzliche Verbindung vorgesehen wird, die bei Verschiebung des Ringflansches in distaler Richtung die Hülse 33 mitnimmt. Eine solche Verbindung ist in dem Ausführungsbeispiel der Figuren 6 und 7 nicht vorgesehen, dort liegt der Ringflansch 37 lediglich an der Hülse 33 an und kann diese bei einer axialen Verschiebung in proximaler Richtung verschieben.

## Patentansprüche

1. Schraubendreher (1) für Knochenschrauben (6) mit einem Griffteil (2) und einem daran drehfest gehaltenen Schaft (3), dessen freies Ende (4) einen unrunden Querschnitt aufweist und formschlüssig in eine unrunde Aufnahmeöffnung (5) im Kopf einer Knochenschraube (6) einsetzbar ist, wobei im Schaft (3) mindestens ein Spreizelement (11) in Längsrichtung verschiebbar gelagert ist, welches im Bereich des freien Endes (4) des Schaftes (3) bei seiner Verschiebung in Längsrichtung an einer Aufgleitfläche (10) derart entlanggleitet, daß es über die Kontur des Schaftes (3) seitlich hervortritt, **dadurch gekennzeichnet, daß** das mindestens eine Spreizelement (11) mittels eines elastischen Federelements (16; 35) in Richtung auf das freie Ende (4) des Schaftes (3) vorgespannt ist und gegen die Wirkung des Federelements (16; 35) im Schaft (3) zurückziehbar ist, bis es nicht mehr über die Kontur des Schaftes (3) seitlich hervorsteht.

2. Schraubendreher nach Anspruch 1, **dadurch gekennzeichnet, daß** ein die Vorschubbewegung des mindestens einen Spreizelements (11) in Richtung auf das freie Ende (4) des Schaftes (3) begrenzender Anschlag vorgesehen ist.

3. Schraubendreher nach Anspruch 1 oder 2, **dadurch gekennzeichnet, daß** das mindestens eine Spreizelement über ein Übertragungsglied (11) mit einer Vorschubeinrichtung verbunden ist, die sich am Griffteil (2) befindet, und daß das elastische Federelement (16) Teil der Vorschubeinrichtung ist.

4. Schraubendreher nach Anspruch 3, **dadurch gekennzeichnet, daß** das Übertragungsglied mindestens einen Stab (11) umfaßt, der in mindestens eine seitlich offene, sich über den gesamten Schaft (3) bis zum Griffteil (2) erstreckende Längsnut (7) eingelegt ist.

5. Schraubendreher nach einem der Ansprüche 3 oder 4, **dadurch gekennzeichnet, daß** die Vorschubeinrichtung eine mit dem mindestens einen Spreizelement (11) verbundene, auf dem Schaft (3) verschiebbare Hülse (13, 33) umfaßt.

6. Schraubendreher nach Anspruch 5, **dadurch gekennzeichnet, daß** die Hülse (13) seitlich vorstehende Griffflächen (14) trägt.

7. Schraubendreher nach Anspruch 6, **dadurch gekennzeichnet, daß** die Griffflächen durch einen radial abstehenden Ringflansch (14) gebildet werden.

8. Schraubendreher nach Anspruch 5, **dadurch gekennzeichnet, daß** zur Verschiebung der Hülse (33) ein Schwenkelement (34) vorgesehen ist, welches an einem Ende schwenkbar am Schaft (3) gelagert ist und um diese Lagerstelle (B) gegen den Schaft (3) verschwenkbar ist, und daß das Schwenkelement (34) einen Mitnehmer (37) aufweist, der beim Verschwenken des Schwenkelementes (34) gegen den Schaft (3) die Hülse (33) entgegen der Wirkung des Federelementes (16; 35) verschiebt.

9. Schraubendreher nach Anspruch 8, **dadurch gekennzeichnet, daß** das Schwenkelement (34) als die Hülse (33) umgebende Schwenkhülse ausgebildet ist.

10. Schraubendreher nach Anspruch 8 oder 9, **dadurch gekennzeichnet, daß** das Schwenkelement (34) mittels einer elastisch verformbaren Halterung (35) am Schaft (3) gehalten ist und die Lagerstelle (B) durch ein Abstützelement (37) des Schwenkelements (34) am Schaft (3) gebildet wird.

11. Schraubendreher nach Anspruch 10, **dadurch gekennzeichnet, daß** die Halterung (35) durch einen den Schaft (3) konzentrisch umgebenden Balg gebildet wird.

12. Schraubendreher nach Anspruch 10 oder 11, **dadurch gekennzeichnet, daß** das Abstützelement durch einen an der Innenseite der Schwenkhülse (34) nach innen vorspringenden Ringflansch (37) gebildet wird.

13. Schraubendreher nach Anspruch 12, **dadurch gekennzeichnet, daß** der Ringflansch (37) den Mitnehmer bildet.

14. Schraubendreher nach einem der Ansprüche 11 bis 13, **dadurch gekennzeichnet, daß** das Federelement durch den elastischen Balg (35) gebildet wird.

15. Schraubendreher nach einem der Ansprüche 5 bis 13, **dadurch gekennzeichnet, daß** die Hülse (13; 33) den Schaft (3) im Abstand umgibt und daß in einem Ringraum (15) zwischen Hülse (13; 33) und Schaft (3) als Federelement eine den Schaft (3) umgebende, sich an dem Schaft (3) und an dem mindestens einen Spreizelement (11) abstützende Schraubenfeder (16) angeordnet ist.

16. Schraubendreher nach einem der Ansprüche 4 bis 15, **dadurch gekennzeichnet, daß** die mindestens eine Längsnut (7) und der mindestens eine Stab (11) so dimensioniert sind, daß der mindestens eine Stab (11) zumindest im Bereich des freien Endes (4) des Schaftes (3) spielfrei in die mindestens eine Längsnut (7) eingreift.

17. Schraubendreher nach Anspruch 16, **dadurch gekennzeichnet, daß** die mindestens eine Längsnut (7) und der mindestens eine Stab (11) zumindest im Bereich des freien Endes (4) des Schaftes (3) zum Nutboden (9) hin konvergierende, ebene Seitenflächen (26, 27; 29, 30) gleicher Neigung aufweisen.

18. Schraubendreher nach Anspruch 17, **dadurch gekennzeichnet, daß** der mindestens eine Stab (11) zumindest im Bereich des freien Endes (4) des Schaftes (3) an seiner dem Nutboden (9) zugewandten Unterseite (28) breiter ist als der Nutboden (9), so daß beim Einlegen des mindestens einen Stabes (11) in die mindestens eine Längsnut (7) zwischen der Unterseite (28) und dem Nutboden (9) ein Abstand verbleibt.

19. Schraubendreher nach einen der Ansprüche 17 oder 18, **dadurch gekennzeichnet, daß** die Seitenwände (26, 27) der mindestens einen Längsnut (7) zumindest im Bereich des freien Endes (4) des Schaftes (3) über eine Abrundung (31) in den Nutboden (9) übergehen.

20. Schraubendreher nach Anspruch 19, **dadurch gekennzeichnet, daß** der Nutboden (9) zumindest im Bereich des freien Endes (4) des Schaftes (3) einen bogenförmigen Querschnitt aufweist.

21. Schraubendreher nach einem der Ansprüche 19 oder 20, **dadurch gekennzeichnet, daß** die Seitenwände (29, 30) des mindestens einen Stabes (11) zumindest im Bereich des freien Endes (4) des Schaftes (3) über eine Abrundung (32) in die Unterseite (28) übergehen.

22. Schraubendreher nach einem der Ansprüche 20 oder 21, **dadurch gekennzeichnet, daß** die Unterseite (28) des mindestens einen Stabes (11) zumindest im Bereich des freien Endes (4) des Schaftes (3) einen bogenförmigen Querschnitt aufweist.

23. Schraubendreher nach einem der voranstehenden Ansprüche, **dadurch gekennzeichnet, daß** der unrunde Querschnitt der Aufnahmeöffnung (5) und der unrunde Querschnitt des freien Endes (4) des Schaftes (3) derart ausgebildet sind, daß der Schaft (3) durch das Spreizelement (11) in zwei voneinander getrennten Bereichen (24, 25) der Aufnahmeöffnung (5) gegen eine Innenwand der Aufnahmeöffnung (5) angedrückt wird.

24. Schraubendreher nach Anspruch 23, **dadurch gekennzeichnet, daß** die Aufnahmeöffnung (5) die Form eines regelmäßigen symmetrischen Sechsecks oder Achtecks aufweist und der Schaft (3) im wesentlichen komplementär geformt ist, wobei die dem mindestens einen Spreizelement (11) gegenüberliegende Seitenfläche (20) des Schaftes (3) geringfügig radial nach innen zurückgesetzt ist.

## Claims

1. Screwdriver (1) for bone screws (6), having a handle (2) and rotationally fixedly held thereon a shaft (3), the free end (4) of which has a non-circular cross section and is insertable in a positively locked manner in a non-circular receiving opening (5) in the head of a bone screw (6), at least one expander element (11) being mounted so as to be displaceable in the longitudinal direction in the shaft (3) and so as to slide, when displaced in the longitudinal direction, along a slide surface (10) in the area of the free end (4) of the shaft (3) in such a way that it projects laterally over the contour of the shaft (3), **characterized in that** the at least one expander element (11) is pretensioned by an elastic spring element (16; 35) in the direction towards the free end (4) of the shaft (3) and is retractable against the action of the spring element (16; 35) in the shaft (3) until it no longer projects laterally over the contour of the shaft (3).

2. Screwdriver in accordance with claim 1, **characterized in that** a stop is provided for limiting the advancing movement of the at least one expander element (11) in the direction towards the free end (4) of the shaft (3).

3. Screwdriver in accordance with claim 1 or 2, **characterized in that** the at least one expander element is connected via a transmission member (11) to an advancing device located on the handle (2), and **in that** the elastic spring element (16) is part of the advancing device.

4. Screwdriver in accordance with claim 3, **characterized in that** the transmission member comprises at least one bar (11) which is placed in at least one longitudinal groove (7) which is laterally open and extends over the entire shaft (3) as far as the handle (2).

5. Screwdriver in accordance with claim 3 or 4, **characterized in that** the advancing device comprises a sleeve (13, 33) which is connected to the at least one expander element (11) and is displaceable on the shaft (3).

6. Screwdriver in accordance with claim 5, **characterized in that** the sleeve (13) carries gripping surfaces (14) which project laterally.

7. Screwdriver in accordance with claim 6, **characterized in that** the gripping surfaces are formed by a radially projecting ring flange (14).

8. Screwdriver in accordance with claim 5, **characterized in that** a swivel element (34) which is mounted on the shaft (3) for swivel movement at one end and is able to swivel about this bearing point (B) against the shaft (3) is provided for displacing the sleeve (33), and **in that** the swivel element (34) comprises a driver (37) which displaces the sleeve (33) against the action of the spring element (16; 35) when the swivel element (34) is swiveled against the shaft (3).

9. Screwdriver in accordance with claim 8, **characterized in that** the swivel element (34) is configured as a swivel sleeve surrounding the sleeve (33).

10. Screwdriver in accordance with claim 8 or 9, **characterized in that** the swivel element (34) is held by an elastically deformable holder (35) on the shaft (3), and the bearing point (B) is formed by a supporting element (37) of the swivel element (34) on the shaft (3).

11. Screwdriver in accordance with claim 10, **characterized in that** the holder (35) is formed by a bellows concentrically surrounding the shaft (3).

12. Screwdriver in accordance with claim 10 or 11, **characterized in that** the supporting element is formed by a ring flange (37) projecting inwardly on the inner side of the swivel sleeve (34).

13. Screwdriver in accordance with claim 12, **characterized in that** the ring flange (37) forms the driver.

14. Screwdriver in accordance with any one of claims 11 to 13, **characterized in that** the spring element is formed by the elastic bellows (35).

15. Screwdriver in accordance with any one of claims 5 to 13, **characterized in that** the sleeve (13; 33) surrounds the shaft (3) in spaced relation thereto, and **in that** a helical spring (16) which surrounds the shaft (3) and is supported on the shaft (3) and on the at least one expander element (11) is arranged as spring element in a ring space (15) between sleeve (13; 33) and shaft (3).

16. Screwdriver in accordance with any one of claims 4 to 15, **characterized in that** the at least one longitudinal groove (7) and the at least one bar (11) are of such dimensions that, at least in the area of the free end (4) of the shaft (3), the at least one bar (11) engages the at least one longitudinal groove (7) without play.

17. Screwdriver in accordance with claim 16, **characterized in that** the at least one longitudinal groove (7) and the at least one bar (11) have, at least in the area of the free end (4) of the shaft (3), planar side surfaces (26, 27; 29, 30) having the same inclination and converging towards the bottom (9) of the groove.

18. Screwdriver in accordance with claim 17, **characterized in that** the at least one bar (11) is, at least in the area of the free end (4) of the shaft (3), wider on its underside (28) facing the bottom (9) of the groove than the bottom (9) of the groove, so that a spacing remains between the underside (28) and the bottom (9) of the groove when inserting the at least one bar (11) into the at least one longitudinal groove (7).

19. Screwdriver in accordance with claim 17 or 18, **characterized in that** the side walls (26, 27) of the at least one longitudinal groove (7) continue, at least in the area of the free end (4) of the shaft (3), via a rounding-off (31) into the bottom (9) of the groove.

20. Screwdriver in accordance with claim 19, **characterized in that** the bottom (9) of the groove has, at least in the area of the free end (4) of the shaft (3), an arcuate cross section.

21. Screwdriver in accordance with claim 19 or 20, **characterized in that** the side walls (29, 30) of the at least one bar (11) continue, at least in the area of the free end (4) of the shaft (3), via a rounding-off (32) into the underside (28).

22. Screwdriver in accordance with claim 20 or 21, **characterized in that** the underside (28) of the at least one bar (11) has, at least in the area of the free end (4) of the shaft (3), an arcuate cross section.

23. Screwdriver in accordance with any one of the preceding claims, **characterized in that** the non-circular cross section of the receiving opening (5) and the non-circular cross section of the free end (4) of the shaft (3) are configured such that the shaft (3) is pressed by the expander element (11) in two areas (24, 25) of the receiving opening (5) that are separate from one another against an inside wall of the receiving opening (5).

24. Screwdriver in accordance with claim 23, **characterized in that** the receiving opening (5) has the shape of a regular symmetrical hexagon or octagon, and the shaft (3) is of substantially complementary shape, and the side surface (20) of the shaft (3) that lies opposite the at least one expander element (11) is set back radially inwardly to a slight extent.

## Revendications

1. Tournevis (1) pour vis à os (6) comprenant une partie formant poignée (2) laquelle porte un corps allongé (3) bloqué contre la rotation dont l'extrémité libre (4) présente une section transversale non ronde et qui peut être inséré par complémentarité de formes dans une ouverture de réception (5) non ronde de la tête d'une vis à os (6), tournevis pour lequel dans le corps allongé (3) est monté coulissant dans la direction longitudinale, au moins un élément d'expansion (11), qui, dans la zone de l'extrémité libre (4) du corps allongé (3), lors de son coulissement dans la direction longitudinale, glisse sur une surface de rampe (10) de manière à dépasser latéralement du contour du corps allongé (3), **caractérisé en ce que** ledit élément ou les éléments d'expansion (11) est ou sont précontraints, au moyen d'un élément de ressort (16 ; 35) élastique, en direction de l'extrémité libre (4) du corps allongé (3), et peut ou peuvent être rétractés dans le corps allongé (3) à l'encontre de l'action de l'élément de ressort (16 ; 35), jusqu'à ce qu'ils ne dépassent plus latéralement du contour du corps allongé (3).

2. Tournevis selon la revendication 1, **caractérisé en ce qu'**il est prévu une butée limitant le mouvement d'avance dudit élément ou des éléments d'expansion (11) en direction de l'extrémité libre (4) du corps allongé (3).

3. Tournevis selon la revendication 1 ou 2, **caractérisé en ce que** ledit élément ou les éléments d'expansion est ou sont reliés, par l'intermédiaire d'un organe de transmission (11), à un dispositif d'avance qui se trouve dans la partie formant poignée (2), et **en ce que** l'élément de ressort (16) élastique fait partie du dispositif d'avance.

4. Tournevis selon la revendication 3, **caractérisé en ce que** l'organe de transmission comprend au moins une tige (11) qui est placée dans au moins une rainure longitudinale (7) ouverte latéralement et s'étendant sur la totalité du corps allongé (3) jusqu'à la partie formant poignée (2).

5. Tournevis selon l'une des revendications 3 ou 4, **caractérisé en ce que** le dispositif d'avance comprend une douille (13, 33) pouvant coulisser sur le corps allongé (3) et reliée audit élément ou aux éléments d'expansion (11).

6. Tournevis selon la revendication 5, **caractérisé en ce que** la douille (13) porte des surfaces de préhension (14) en saillie latérale.

7. Tournevis selon la revendication 6, **caractérisé en ce que** les surfaces de préhension sont formées par un flasque annulaire (14) en saillie radiale.

8. Tournevis selon la revendication 5, **caractérisé en ce que** pour le coulissement de la douille (33) il est prévu un élément pivotant (34), qui est monté pivotant, à une extrémité, sur le corps allongé (3), et peut pivoter autour de ce point de palier (B) par rapport au corps allongé (3), et **en ce que** l'élément pivotant (34) comporte un entraîneur (37) qui, lors du pivotement de l'élément pivotant (34) par rapport au corps allongé (3), fait coulisser la douille (33) à l'encontre de l'action de l'élément de ressort (16 ; 35).

9. Tournevis selon la revendication 8, **caractérisé en ce que** l'élément pivotant (34) est réalisé en tant que douille pivotante entourant la douille (33).

10. Tournevis selon la revendication 8 ou 9, **caractérisé en ce que** l'élément pivotant (34) est supporté sur le corps allongé (3) au moyen d'un élément de support (35) déformable élastiquement, et le point de palier (B) est formé par un élément d'appui (37) de l'élément pivotant (34) sur le corps allongé (3).

11. Tournevis selon la revendication 10, **caractérisé en ce que** l'élément de support (35) est formé par un soufflet entourant de manière concentrique le corps allongé (3).

12. Tournevis selon la revendication 10 ou 11, **caractérisé en ce que** l'élément d'appui est formé par un flasque annulaire (37) faisant saillie vers l'intérieur, sur le côté intérieur de la douille pivotante (34).

13. Tournevis selon la revendication 12, **caractérisé en ce que** le flasque annulaire (37) forme l'entraîneur.

14. Tournevis selon l'une des revendications 11 à 13, **caractérisé en ce que** l'élément de ressort est formé par le soufflet élastique (35).

15. Tournevis selon l'une des revendications 5 à 13, **caractérisé en ce que** la douille (13 ; 33) entoure le corps allongé (3) à distance, et **en ce que** dans un espace annulaire (15) entre la douille (13 ; 33) et le corps allongé (3), est agencé en guise d'élément de ressort, un ressort hélicoïdal (16) qui entoure le corps allongé (3), et s'appuie sur le corps allongé (3) et sur ledit élément ou les éléments d'expansion (11).

16. Tournevis selon l'une des revendications 4 à 15, **caractérisé en ce que** ladite une rainure ou les rainures longitudinales (7) et ladite une tige ou les tiges (11) sont dimensionnées de façon telle, que ladite tige ou les tiges (11) s'engagent sans jeu, au moins dans la zone de l'extrémité libre (4) du corps allongé (3), dans ladite rainure ou les rainures longitudinales (7).

17. Tournevis selon la revendication 16, **caractérisé en ce que** ladite une rainure ou les rainures longitudinales (7) et ladite une tige ou les tiges (11) présentent, au moins dans la zone de l'extrémité libre (4) du corps allongé (3), des surfaces latérales planes (26, 27 ; 29, 30) de même inclinaison, qui convergent vers le fond de rainure (9).

18. Tournevis selon la revendication 17, **caractérisé en ce que** ladite une tige ou les tiges (11) est ou sont, au niveau de leur côté inférieur (28) dirigé vers le fond de rainure (9), au moins dans la zone de l'extrémité libre (4) du corps allongé (3), plus larges que le fond de rainure (9), de sorte que lors de l'insertion de ladite une tige ou des tiges (11) dans ladite rainure ou les rainures longitudinales (7), il reste une distance d'espacement entre le côté inférieur (28) et le fond de rainure (9).

19. Tournevis selon l'une des revendications 17 ou 18, **caractérisé en ce que** les parois latérales (26, 27) de ladite une rainure ou des rainures longitudinales (7) se raccordent, au moins dans la zone de l'extrémité libre (4) du corps allongé (3), au fond de rainure (9), par l'intermédiaire d'un arrondi (31).

20. Tournevis selon la revendication 19, **caractérisé en ce que** le fond de rainure (9) présente, au moins dans la zone de l'extrémité libre (4) du corps allongé (3), une section transversale en forme d'arc.

21. Tournevis selon l'une des revendications 19 ou 20, **caractérisé en ce que** les parois latérales (29, 30) de ladite une tige ou des tiges (11) se raccordent, au moins dans la zone de l'extrémité libre (4) du corps allongé (3), au côté inférieur (28), par l'intermédiaire d'un arrondi (32).

22. Tournevis selon l'une des revendications 20 ou 21, **caractérisé en ce que** le côté inférieur (28) de ladite une tige ou des tiges (11) présente, au moins dans la zone de l'extrémité libre (4) du corps allongé (3), une section transversale en forme d'arc.

23. Tournevis selon l'une des revendications précédentes, **caractérisé en ce que** la section transversale non ronde de l'ouverture de réception (5) et la section transversale non ronde de l'extrémité libre (4) du corps allongé (3) sont réalisées de façon que le corps allongé (3) soit pressé par l'élément d'expansion (11) contre une paroi intérieure de l'ouverture de réception (5), au niveau de deux zones (24, 25) de l'ouverture de réception (5), séparées l'une de l'autre.

24. Tournevis selon la revendication 23, **caractérisé en ce que** l'ouverture de réception (5) présente la forme d'un hexagone ou octogone symétrique régulier, et le corps allongé (3) est de forme sensiblement complémentaire, la face latérale (20) du corps allongé (3), qui est opposée audit élément ou aux éléments d'expansion (11), étant légèrement décalée en retrait, radialement vers l'intérieur.
